# EUROPÄISCHE PATENTSCHRIFT

(11) **EP 4 198 008 B1**
(45) Veröffentlichungstag und Bekanntmachung des Hinweises auf die Patenterteilung: **31.07.2024**
(21) Anmeldenummer: 21215350.6
(22) Anmeldetag: 17.12.2021
(51) Int. Cl.: C07C 45/50, C07C 47/02, C07C 47/12, C07C 47/228

(54) **VERFAHREN ZUR HYDROFORMYLIERUNG VON OLEFINEN UNTER EINSATZ VON PT UND IOD**
METHOD FOR HYDROFORMYLATION OF OLEFINS USING PT AND IODINE
PROCÉDÉ D'HYDROFORMYLATION D'OLÉFINES À L'AIDE DU PT ET DE L'IODE

(43) Veröffentlichungstag der Anmeldung: 21.06.2023
(73) Patentinhaber: Evonik Oxeno GmbH & Co. KG, 45772 Marl (DE)
(72) Erfinder: SCHNEIDER, Carolin, 40789 Monheim am Rhein (DE); JACKSTELL, Ralf, 18106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- PETOCZ G ET AL: "Xantphos as cis- and trans-chelating ligand in square-planar platinum(II) complexes. Hydroformylation of styrene with platinum-xantphos-tin(II)chloride system", JOURNAL OF ORGANOMETALLIC CHEMISTRY, ELSEVIER, AMSTERDAM, NL, vol. 689, no. 7, 1 April 2004 (2004-04-01), pages 1188 - 1193, XP004496421, ISSN: 0022-328X, DOI: 10.1016/J.JORGANCHEM.2003.10.045

## Beschreibung

Die vorliegende Erfindung betrifft ein Verfahren zur Hydroformylierung von Olefinen unter Einsatz von Pt und lod.

In G. Petöcz et al., Journal of Organometallic Chemistry 689 (2004), 1188-1193 wird das Platin-Xantphos-Zinn(II)chlorid System als aktiver Katalysator in der Hydroformylierung von Styrol beschrieben.

In C. Botteghi et al., Journal of Molecular Catalysis A: Chemical 200, (2003), 147-156 wird der Einsatz von Pt(Xantphos)Cl₂ zur Hydroformylierung von 2-Tosyloxystyrol beschrieben.

Der vorliegende Erfindung lag die Aufgabe zugrunde, eine neues Hydroformylierungsverfahren bereitzustellen. Das Verfahren soll hierbei eine gegenüber dem aus dem Stand der Technik unter Einsatz von Pt(Xantphos)Cl₂ bekannten Verfahren gesteigerte Ausbeute liefern.

Diese Aufgabe wird gelöst durch ein Verfahren gemäß Anspruch 1.

Verfahren umfassend die Verfahrensschritte:
a) Vorlegen eines Olefins;
b) Zugabe einer Verbindung gemäß der Formel (**I**): 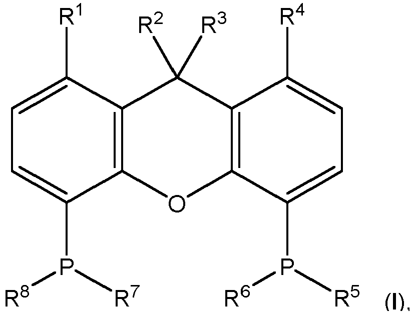 wobei R¹, R², R³, R⁴, R⁵, R⁶, R⁷, R⁸ ausgewählt sind aus: -H, -(C₁-C₁₂)-Alkyl, -(C₆-C₂₀)-Aryl; und für den Fall, dass R¹, R², R³, R⁴, R⁵, R⁶, R⁷, R⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
c) Zugabe einer Pt-Verbindung, welche zur Ausbildung eines Komplexes befähigt ist;
d) Zugabe einer lod-Verbindung;
e) Zuführen von CO und H₂;
f) Erwärmen des Reaktionsgemisches aus a) bis e), wobei das Olefin zu einem Aldehyd umgesetzt wird.

Hierbei können die Verfahrensschritte a) bis e) in beliebiger Reihenfolge erfolgen. Üblicherweise erfolgt die Zugabe von CO und H₂ jedoch, nachdem die Reaktionspartner in den Schritten a) bis d) vorgelegt wurden.

Hierbei können die Verfahrensschritte c) und d) in einem Schritt, durch Zugabe von Ptl₂, erfolgen.

In einer bevorzugten Variante des Verfahrens erfolgt die Zugabe der Pt-Verbindung und der lod-Verbindung in einem Schritt, durch Zugabe von Ptl₂.

Der Begriff (C₁-C₁₂)-Alkyl umfasst geradkettige und verzweigte Alkylgruppen mit 1 bis 12 Kohlenstoffatomen. Vorzugsweise handelt es sich dabei um (C₁-C₈)-Alkylgruppen, besonders bevorzugt (C₁-C₆)-Alkyl, am meisten bevorzugt (C₁-C₄)-Alkyl.

Geeignete (C₁-C₁₂)-Alkylgruppen sind insbesondere Methyl, Ethyl, Propyl, Isopropyl, n-Butyl, iso-Butyl, sec-Butyl, tert-Butyl, n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,1-Dimethylbutyl, 1,2-Dimethylbutyl, 2,2-Dimethylbutyl, 1,3-Dimethylbutyl, 2,3-Dimethylbutyl, 3,3-Dimethylbutyl, 1,1,2-Trimethylpropyl, 1,2,2-Trimethylpropyl, 1-Ethylbutyl, 1-Ethyl-2-methylpropyl, n-Heptyl, 2-Heptyl, 3-Heptyl, 2-Ethylpentyl, 1-Propylbutyl, n-Octyl, 2-Ethylhexyl, 2-Propylheptyl, Nonyl, Decyl.

Der Begriff (C₆-C₂₀)-Aryl umfasst mono- oder polycyclische aromatische Kohlenwasserstoffreste mit 6 bis 20 Kohlenstoffatomen. Vorzugsweise handelt es sich dabei um (C₆-C₁₄)-Aryl, besonders bevorzugt (C₆-C₁₀)-Aryl.

Geeignete (C₆-C₂₀)-Arylgruppen sind insbesondere Phenyl, Naphthyl, Indenyl, Fluorenyl, Anthracenyl, Phenanthrenyl, Naphthacenyl, Chrysenyl, Pyrenyl, Coronenyl. Bevorzugte (C₆-C₂₀)-Arylgruppen sind Phenyl, Naphthyl und Antracenyl.

In einer Variante des Verfahrens sind R², R³, R⁵, R⁶, R⁷, R⁸ ausgewählt aus: -(C₁-C₁₂)-Alkyl, - (C₆-C₂₀)-Aryl.

In einer Variante des Verfahrens stehen R⁵, R⁶, R⁷, R⁸ für -(C₆-C₂₀)-Aryl.

In einer Variante des Verfahrens stehen R⁵, R⁶, R⁷, R⁸ für -Ph

In einer Variante des Verfahrens stehen R² und R³ für -(C₁-C₁₂)-Alkyl.

In einer Variante des Verfahrens stehen R² und R³ für -CH₃.

In einer Variante des Verfahrens stehen R¹ und R⁴ für -H.

In einer Variante des Verfahrens weisen die Verbindung (**I**) die Struktur (1) auf:

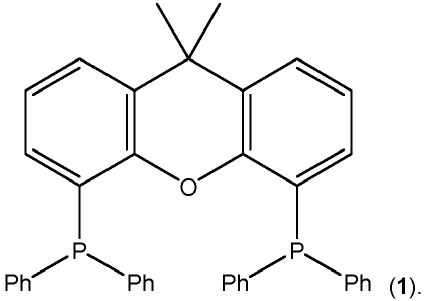

In einer Variante des Verfahrens ist die Pt-Verbindung ausgewählt aus: Pt(II)I₂, Pt(IV)I₄, Diphenyl(1,5-COD)Pt(II), Pt(II)(acac)₂, Pt(0)(PPh₃)₄, Pt(0)(DVTS)-Lösung (CAS: 68478-92-2), Pt(0)(Ethylen)(PPh₃)₂, Tris(benzylidenaceton)Pt(0), Pt(II)(OAC)₂-Lösung, Pt(0)(t-Bu)₂, Pt(II)(COD)Me₂, Pt(II)(COD)l₂, Pt(IV)IMe₃, Pt(II)(Hexafluoroacetylacetonat)₂.

In einer Variante des Verfahrens ist die Pt-Verbindung ausgewählt aus: Pt(II)I₂, Pt(II)(acac)₂.

In einer Variante des Verfahrens ist die lod-Verbindung ausgewählt aus: Alkalihalogenid, Erdalkalihalogenid, NH₄X, Alkylammoniumhalogenid, Dialkylhalogenid, Trialkylhalogenid, Tetraalkylhalogenid, Cycloalkylammoniumhalogenid.

In einer Variante des Verfahrens ist die lod-Verbindung ausgewählt aus: Pt(II)I₂, Lil.

In einer Variante des Verfahrens wird die lod-Verbindung in einer Menge zugegeben, gemessen in Äquivalenten bezogen auf Pt, welche im Bereich von 0.1 bis 10 liegt.

In einer Variante des Verfahrens umfasst dieses den zusätzlichen Verfahrensschritt e`): e') Zugabe eines Lösungsmittels.

In einer Variante des Verfahrens ist das Lösungsmittel ausgewählt aus: THF, DCM, ACN, Heptan, DMF, Toluol, Texanol, Pentan, Hexan, Octan, Isooctan, Decan, Dodecan, Cyclohexan, Benzen, Xylene, Marlotherm, Propylencarbonat, MTBE, Diglyme, Triglyme, Diethylether, Dioxan, Isopropanol, Tertbutanol, Isononanol, Isobutanol, Isopentanol, Ethylacetat.

In einer Variante des Verfahrens ist das Lösungsmittel ausgewählt aus: THF, DCM, ACN, Heptan, DMF, Toluol, Texanol.

In einer Variante des Verfahrens erfolgt das Zuführen von CO und H₂ bei einem Druck in einem Bereich von 1 MPa (10 bar) bis 6 MPa (60 bar).

In einer Variante des Verfahrens erfolgt das Zuführen von CO und H₂ bei einem Druck in einem Bereich von 1 MPa (20 bar) bis 6 MPa (50 bar).

In einer Variante des Verfahrens erfolgt das Erwärmen auf eine Temperatur im Bereich von 25°C bis 150°C.

In einer Variante des Verfahrens erfolgt das Erwärmen auf eine Temperatur im Bereich von 30 °C bis 130°C.

In einer Variante des Verfahrens ist das Olefin ausgewählt aus: Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

Im Folgenden soll die Erfindung anhand von Ausführungsbeispielen näher erläutert werden.

### Versuchsbeschreibung

Ein Vial wurde mit PtX₂ (X = Halogen), Ligand, und einem im Ofen getrockneten Rührstab bestückt. Dann wird das Vial mit Septum (PTFE-beschichteter StyrolButadien-Kautschuk) und Phenolharzdeckel verschlossen. Das Vial wird dreimal evakuiert und mit Argon wieder befüllt. Mit einer Spritze wurden Toluol und Olefin in das Vial gegeben. Das Vial wurde in eine Legierungsplatte gestellt, die in einen Autoklav der Reihe 4560 von Parr Instruments unter Argon Atmosphäre überführt wurde. Nach dreimaligem Spülen des Autoklaven mit CO/H₂ wurde der Synthesegasdruck auf 40 bar bei Raumtemperatur erhöht. Die Reaktion wurde 20h / 18 h bei 120 °C / 80 °C durchgeführt. Nach Beendigung der Reaktion wurde der Autoklav auf Raumtemperatur abgekühlt und vorsichtig entspannt. Der Ausbeute und Selektivität wurden durch GC-Analyse bestimmt.

### Variation des Metalls

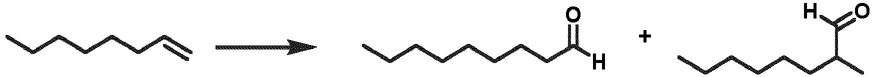

### Reaktionsbedingungen:

20 mmol 1-Octen, 0.1 mol% Metall, 2.2 Äquivalente Xantphos (1), Lösungsmittel: Toluol, p(CO/H₂): 40 bar, T: 80 °C, t: 20 h.

### Ausbeuten:

Ptl₂: 99%
Pdl₂: 0%

### Variation des Halogens (2-Octen)

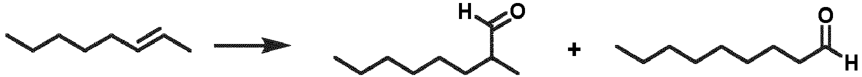

### Reaktionsbedingungen:

20 mmol 2-Octen, 1.0 mol% Pt, 1.1 Äquivalente Xantphos (1), Lösungsmittel: Toluol, p(CO/H₂): 40 bar, T: 120 °C, t: 20 h.

### Ausbeuten:

Ptl₂: 99%
PtCl₂: 16%

### Variation des Halogens (1-Octen)

### Reaktionsbedingungen:

10.0 mmol 1-Octen, 0.1 mol% PtX₂, 2.2 Äquivalente Ligand, Lösungsmittel: Toluol, p(CO/H₂): 40 bar, T: 120 °C, t: 20 h.

### Ausbeuten:

| Ligand | Halogen | Ausbeute [%] |
|---|---|---|
| 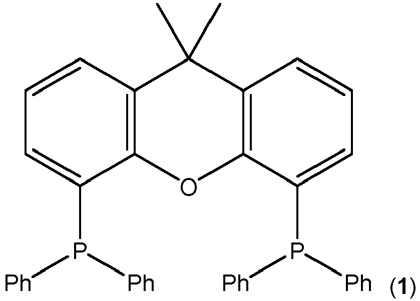 | I / Cl | 99 / 5 |

### Variation des Olefins

### Reaktionsbedingungen:

1.0 mmol Olefin, 0.5 mol% Ptl₂, 2.2 Äquivalente Xantphos (1), Lösungsmittel: Dichlormethan (DCM), p(CO/H₂): 40 bar, T: 80 °C, t: 18 h.

### Ausbeuten:

| Aldehyd | Ausbeute [%] |
|---|---|
| 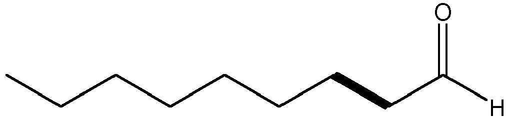 | 99 |
| 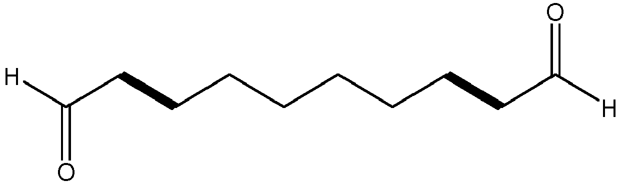 | 99 |
| 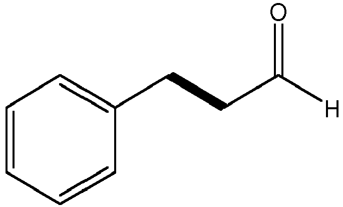 | 99 |
| 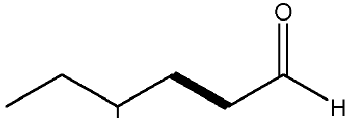 | 99 |

Die fett Markierte C-C-Bindung gibt die Lage der ehemaligen Doppelbindung, also der Doppelbindung im Olefin, an.

### Variation des Liganden und des Halogens

### Reaktionsbedingungen:

1.0 mmol 1-Octen, 0.5 mol% PtX₂, 2.0 Äquivalente Ligand, Lösungsmittel: Toluol, p(CO/H₂): 40 bar, T: 80 °C, t: 18 h.

### Ausbeuten:

| Ligand | Halogen | Ausbeute [%] |
|---|---|---|
| 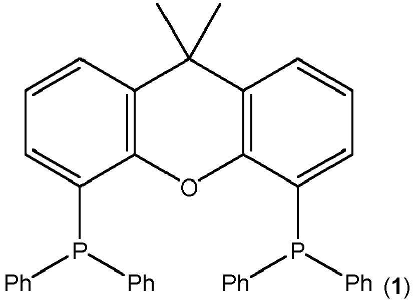 | I / Cl | 99/0 |
| 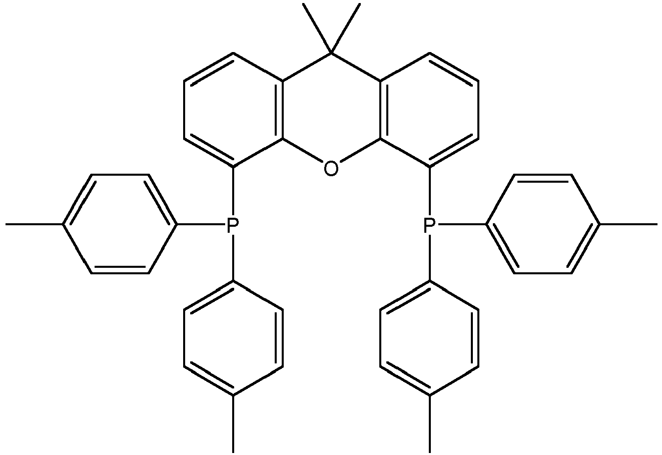 | I / Cl | 99 / 0 |
| 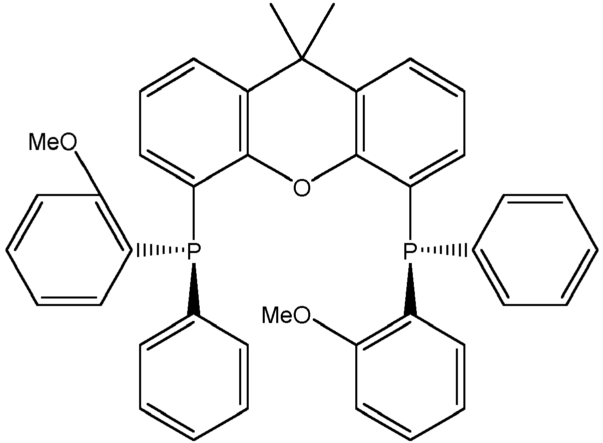 | I / Cl | 99 / < 1 |
| 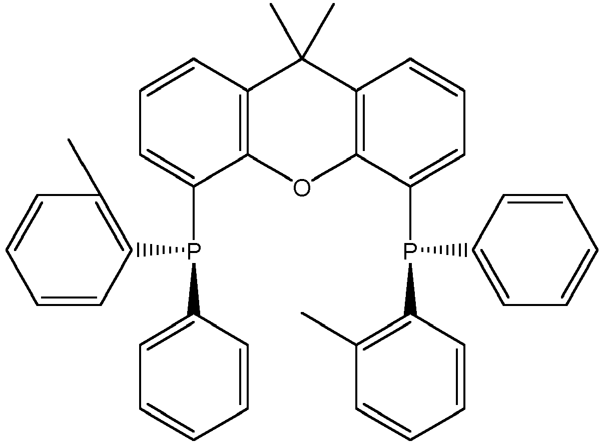 | I / Cl | 95 / 0 |

### Variation der Äquivalente und des Halogens

### Reaktionsbedingungen:

1.0 mmol 1-Octen, 1.0 mol% Pt(acac)₂, LiX (X= Halogen), 2.2 Äquivalente Xantphos (1), Lösungsmittel: Toluol, p(CO/H₂): 40 bar, T: 120 °C, t: 20 h.

| Äquivalente LiX | X | Ausbeute [%] |
|---|---|---|
| 0.5 | I | 99 |
| 1.0 | I | 99 |
| 2.0 | I | 99 |
| 4.0 | I | 99 |
| 4.0 | Cl | 0 |

Wie die Versuchsergebnisse zeigen, wird die Aufgabe durch das erfindungsgemäße Verfahren gelöst. Die Versuche, in denen das Halogen (X) CI ist, sind Vergleichsversuche.

## Patentansprüche

1. Verfahren umfassend die Verfahrensschritte:
a) Vorlegen eines Olefins;
b) Zugabe einer Verbindung gemäß der Formel (**I**): 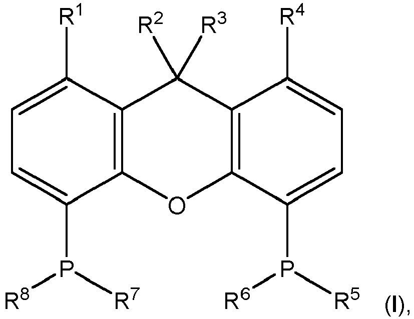 wobei R¹, R², R³, R⁴, R⁵, R⁶, R⁷, R⁸ ausgewählt sind aus: -H, -(C₁-C₁₂)-Alkyl, -(C₆-C₂₀)-Aryl; und für den Fall, dass R¹, R², R³, R⁴, R⁵, R⁶, R⁷, R⁸ für -(C₆-C₂₀)-Aryl stehen, kann der Arylring Substituenten aufweisen, welche ausgewählt sind aus: -(C₁-C₁₂)-Alkyl, -O-(C₁-C₁₂)-Alkyl;
c) Zugabe einer Pt-Verbindung, welche zur Ausbildung eines Komplexes befähigt ist;
d) Zugabe einer lod-Verbindung;
e) Zuführen von CO und H₂;
f) Erwärmen des Reaktionsgemisches aus a) bis e), wobei das Olefin zu einem Aldehyd umgesetzt wird.

2. Verfahren nach Anspruch 1,
wobei R², R³, R⁵, R⁶, R⁷, R⁸ ausgewählt sind aus: -(C₁-C₁₂)-Alkyl, -(C₆-C₂₀)-Aryl.

3. Verfahren nach einem der Ansprüche 1 oder 2,
wobei R⁵, R⁶, R⁷, R⁸ für -(C₆-C₂₀)-Aryl stehen.

4. Verfahren nach einem der Ansprüche 1 bis 3,
wobei R² und R³ für -(C₁-C₁₂)-Alkyl stehen.

5. Verfahren nach einem der Ansprüche 1 bis 4,
wobei R¹ und R⁴ für -H stehen.

6. Verfahren nach einem der Ansprüche 1 bis 5, wobei die Verbindung (**I**) die Struktur (1) aufweist:

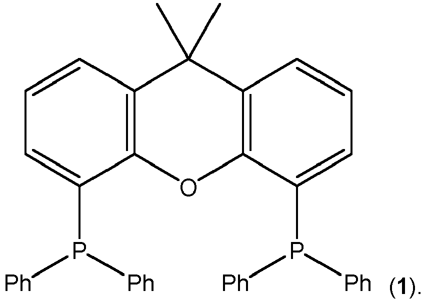

7. Verfahren nach einem der Ansprüche 1 bis 6,
wobei die Pt-Verbindung ausgewählt ist aus: Pt(II)I₂, Pt(IV)I₄, Diphenyl(1,5-COD)Pt(II), Pt(II)(acac)₂, Pt(0)(PPh₃)₄, Pt(0)(DVTS)-Lösung (CAS: 68478-92-2), Pt(0)(Ethylen)(PPh₃)₂, Tris(benzylidenaceton)Pt(0), Pt(II)(OAC)₂-Lösung, Pt(0)(t-Bu)₂, Pt(II)(COD)Me₂, Pt(II)(COD)I₂, Pt(IV)IMe₃, Pt(II)(Hexafluoroacetylacetonat)₂.

8. Verfahren nach einem der Ansprüche 1 bis 7,
wobei die Pt-Verbindung ausgewählt ist aus: Pt(II)I₂, Pt(II)(acac)₂.

9. Verfahren nach einem der Ansprüche 1 bis 8,
wobei die lod-Verbindung ausgewählt ist aus: Pt(II)I₂, Lil.

10. Verfahren nach einem der Ansprüche 1 bis 9,
wobei die lod-Verbindung in einer Menge zugegeben wird, gemessen in Äquivalenten bezogen auf Pt, welche im Bereich von 0.1 bis 10 liegt.

11. Verfahren nach einem der Ansprüche 1 bis 10,
umfassend den zusätzlichen Verfahrensschritt e`):
e') Zugabe eines Lösungsmittels.

12. Verfahren nach Anspruch 11,
wobei das Lösungsmittel ausgewählt ist aus: THF, DCM, ACN, Heptan, DMF, Toluol, Texanol, Pentan, Hexan, Octan, Isooctan, Decan, Dodecan, Cyclohexan, Benzen, Xylene, Marlotherm, Propylencarbonat, MTBE, Diglyme, Triglyme, Diethylether, Dioxan, Isopropanol, Tertbutanol, Isononanol, Isobutanol, Isopentanol, Ethylacetat.

13. Verfahren nach einem der Ansprüche 1 bis 12,
wobei das Zuführen von CO und H₂ bei einem Druck erfolgt in einem Bereich von 1 MPa (10 bar) bis 6 MPa (60 bar).

14. Verfahren nach einem der Ansprüche 1 bis 13,
wobei das Erwärmen auf eine Temperatur erfolgt im Bereich von 25 °C bis 150°C.

15. Verfahren nach einem der Ansprüche 1 bis 14,
wobei das Olefin ausgewählt ist aus: Ethen, Propen, 1-Buten, cis- und/oder trans-2-Buten, iso-Buten, 1,3-Butadien, 1-Penten, cis- und/oder trans-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

## Claims

1. Process comprising the process steps of:
a) initially charging an olefin;
b) adding a compound of formula (I): 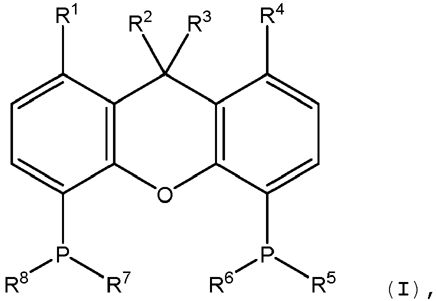
where R¹, R², R³, R⁴, R⁵, R⁶, R⁷, R⁸ are selected from: -H, - (C₁-C₁₂) -alkyl, - (C₆-C₂₀) -aryl;
and, if R¹, R², R³, R⁴, R⁵, R⁶, R⁷, R⁸ are -(C₆-C₂₀)-aryl, the aryl ring may have substituents selected from: -(C₁-C₁₂) -alkyl, -O-(C₁-C₁₂) -alkyl;
c) adding a Pt compound capable of forming a complex;
d) adding an iodine compound;
e) feeding in CO and H₂;
f) heating the reaction mixture from steps a) to e), to convert the olefin to an aldehyde.

2. Process according to Claim 1,
where R², R³, R⁵, R⁶, R⁷, R⁸ are selected from: -(C₁-C₁₂)-alkyl, -(C₆-C₂₀)-aryl.

3. Process according to either of Claims 1 and 2,
where R⁵, R⁶, R⁷, R⁸ are -(C₆-C₂₀)-aryl.

4. Process according to any of Claims 1 to 3,
where R² and R³ are -(C₁-C₁₂) -alkyl.

5. Process according to any of Claims 1 to 4,
where R¹ and R⁴ are -H.

6. Process according to any of Claims 1 to 5,
wherein the compound (I) has the structure (1):

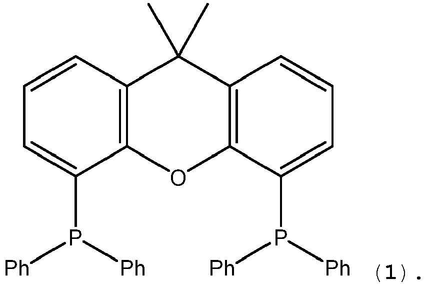

7. Process according to any of Claims 1 to 6,
wherein the Pt compound is selected from: Pt(II)I₂, Pt(IV)I₄, diphenyl (1,5-COD)Pt(II), Pt(II)(acac)₂, Pt(0)(PPh₃)₄, Pt(0)(DVTS) solution (CAS: 68478-92-2), Pt(0)(ethylene)(PPh₃)₂, tris(benzylideneacetone)Pt(0), Pt(II)(OAC)₂ solution, Pt(0)(t-Bu)₂, Pt(II)(COD)Me₂, Pt(II)(COD)I₂, Pt(IV)IMe₃, Pt(II)(hexafluoroacetylacetonate)₂.

8. Process according to any of Claims 1 to 7,
wherein the Pt compound is selected from: Pt(II)I₂, Pt(II)(acac)₂.

9. Process according to any of Claims 1 to 8,
wherein the iodine compound is selected from: Pt(II)I₂, Lil.

10. Process according to any of Claims 1 to 9,
wherein the iodine compound is added in an amount in the range of 0.1 to 10, measured in equivalents based on Pt.

11. Process according to any of Claims 1 to 10,
comprising the additional process step e'):
e') adding a solvent.

12. Process according to Claim 11,
wherein the solvent is selected from: THF, DCM, ACN, heptane, DMF, toluene, texanol, pentane, hexane, octane, isooctane, decane, dodecane, cyclohexane, benzene, xylene, Marlotherm, propylene carbonate, MTBE, diglyme, triglyme, diethyl ether, dioxane, isopropanol, tert-butanol, isononanol, isobutanol, isopentanol, ethyl acetate.

13. Process according to any of Claims 1 to 12,
wherein CO and H₂ are fed in at a pressure in a range from 1 MPa (10 bar) to 6 MPa (60 bar).

14. Process according to any of Claims 1 to 13,
wherein the reaction mixture is heated to a temperature in the range from 25°C to 150°C.

15. Process according to any of Claims 1 to 14,
wherein the olefin is selected from: ethene, propene, 1-butene, cis- and/or trans-2-butene, isobutene, 1,3-butadiene, 1-pentene, cis- and/or trans-2-pentene, 2-methyl-1-butene, 3-methyl-1-butene, 2-methyl-2-butene, hexene, tetramethylethylene, heptene, 1-octene, 2-octene, di-n-butene, or mixtures thereof.

## Revendications

1. Procédé comprenant les étapes suivantes :
a) mise en place d'une oléfine ;
b) addition d'un composé de Formule (1) : 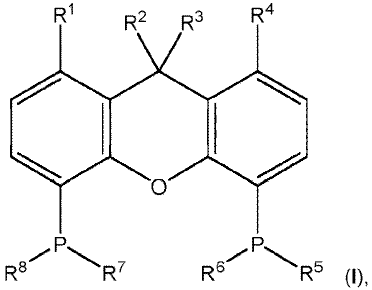
dans laquelle R¹, R², R³, R⁴, R⁵, R⁶, R⁷, R⁸ sont choisis parmi -H, un -alkyle en C₁-C₁₂, un -aryle en C₆-C₂₀ ;
et, dans le cas dans lequel R¹, R², R³, R⁴, R⁵, R⁶, R⁷, R⁸ représentent un -aryle en C₆-C₂₀, le cycle aryle peut présenter des substituants qui sont choisis parmi un -alkyle en C₁-C₁₂, un -O-alkyle en C₁-C₁₂ ;
c) addition d'un composé de Pt qui permet la formation d'un complexe ;
d) addition d'un composé de l'iode ;
e) amenée de CO et de H₂ ;
f) chauffage du mélange réactionnel de a) à e), l'oléfine étant convertie en un aldéhyde.

2. Procédé selon la revendication 1, dans lequel R², R³, R⁵, R⁶, R⁷, R⁸ sont choisis parmi un -alkyle en C₁-C₁₂, un -aryle en C₆-C₂₀.

3. Procédé selon l'une des revendications 1 ou 2, dans lequel R⁵, R⁶, R⁷, R⁸ représentent un -aryle en C₆-C₂₀.

4. Procédé selon l'une des revendications 1 à 3, dans lequel R² et R³ représentent un -alkyle en C₁-C₁₂.

5. Procédé selon l'une des revendications 1 à 4, dans lequel R¹ et R⁴ représentent -H.

6. Procédé selon l'une des revendications 1 à 5, dans lequel le composé (**I**) présente la structure (**1**) :

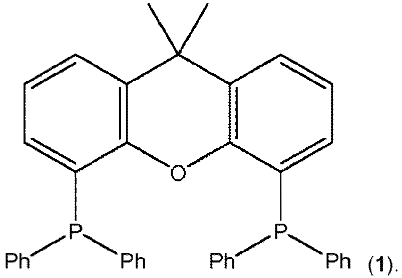

7. Procédé selon l'une des revendications 1 à 6, dans lequel le composé de Pt est choisi parmi Pt(II)I₂, Pt(IV)I₄, diphényl(1,5-COD)Pt(II), Pt(II)(acac)₂, Pt(0)(PPh₃)₄, une solution de Pt(0) (DVTS) (CAS: 68478-92-2), Pt(0)(éthylène)(PPh₃)₂, tris(benzylidèneacétone)Pt(0), une solution de Pt(II)(OAC)₂, Pt(0)(t-Bu)₂, Pt(II)(COD)Me₂, Pt(II)(COD)I₂, Pt(IV)IMe₃, Pt(II)(hexafluoroacétylacétonate)₂.

8. Procédé selon l'une des revendications 1 à 7, dans lequel le composé de Pt est choisi parmi Pt(II)I₂, Pt(II)(acac)₂.

9. Procédé selon l'une des revendications 1 à 8, dans lequel le composé de l'iode est choisi parmi Pt(II)I₂, LiI.

10. Procédé selon l'une des revendications 1 à 9, dans lequel le composé de l'iode est ajouté en une quantité, exprimée en équivalents par rapport à Pt, comprise dans la plage de 0,1 à 10.

11. Procédé selon l'une des revendications 1 à 10, comprenant l'étape supplémentaire e') : e') addition d'un solvant.

12. Procédé selon la revendication 11, dans lequel le solvant est choisi parmi les composés THF, DCM, ACN, heptane, DMF, toluène, texanol, pentane, hexane, octane, isooctane, décane, dodécane, cyclohexane, benzène, xylène, Marlotherm, carbonate de propylène, MTBE, diglyme, triglyme, diéthyléther, dioxane, isopropanol, tert-butanol, isononanol, isobutanol, isopentanol, acétate d'éthyle.

13. Procédé selon l'une des revendications 1 à 12, dans lequel l'amenée de CO et de H₂ a lieu sous une pression dans la plage de 1 MPa (10 bar) à 6 MPa (60 bar).

14. Procédé selon l'une des revendications 1 à 13, dans lequel le chauffage a lieu à une température de 25 °C à 150 °C.

15. Procédé selon l'une des revendications 1 à 14, dans lequel l'oléfine est choisie parmi les composés éthène, propène, 1-butène, cis- et/ou trans-2-butène, iso-butène, 1,3-butadiène, 1-pentène, cis- et/ou trans-2-pentène, 2-méthyl-1-butène, 3-méthyl-1-butène, 2-méthyl-2-butène, hexène, tétraméthyléthylène, heptène, 1-octène, 2-octène, di-n-butène ou les mélanges de ceux-ci.
